# EUROPÄISCHE PATENTSCHRIFT

(11) **EP 2 368 961 B1**
(45) Veröffentlichungstag und Bekanntmachung des Hinweises auf die Patenterteilung: **08.04.2015**
(21) Anmeldenummer: 11000887.7
(22) Anmeldetag: 04.02.2011
(51) Int. Cl.: C09K 8/58, C12N 1/12, C10G 1/04

(54) **Ölgewinnungssubstanz**
Oil extraction substance
Substance pour l'extraction de pétrole

(30) Priorität: 11.02.2010 DE 102010007836
(43) Veröffentlichungstag der Anmeldung: 28.09.2011
(73) Patentinhaber: Maier, Ursula, 8280 Kreuzlingen (CH)
(72) Erfinder: Maier, Ursula, 8280 Kreuzlingen (CH)
(74) Vertreter: Daub, Thomas

(56) Entgegenhaltungen:
- EP-A1- 1 693 084
- US-A- 4 079 544
- US-A- 4 599 117
- US-A1- 2005 255 564

## Beschreibung

Es sind bereits Ölgewinnungssubstanzen bzw. Verfahren zur Herstellung von Ölgewinnungssubstanzen, welche wenigstens einen Bestandteil einer Alge aufweisen, vorgeschlagen worden.

Aus der US 4 599 117 A ist ein Verfahren zur Reinigung ölkontaminierter Feststoffe bekannt. Hierzu werden die ölhaltigen Feststoffe in einen Behälter gegeben, welcher Wasser und ein wasserlösliches Polymer enthält. Als wasserlösliches Polymer kann dabei "Carrageen" verwendet werden, welches aus Algen, insbesondere aus Rotalgen, gewonnen werden kann.

Ferner offenbart die US 4 079 544 A ein Verfahren zur Gewinnung von Erdöl unter Verwendung einer wässrigen Lösung, wobei als Verdicker ein Biopolymer verwendet wird, welches aus Algen gewonnen werden kann.

Des Weiteren ist aus der US 2005/255564 A1 ein Verfahren zur Herstellung von sulfatierten Frucan-Oligosacchariden bekannt. Die sulfatierten Frucan-Oligosaccharide können aus Algen hergestellt werden.

In der EP 1 693 084 A1 ist schließlich ein Verfahren zur Herstellung eines Lipase-Inhibitors aus einer Alge des Typs A. Nodosum zur Behandlung und/oder Vorbeugung von Fettleibigkeit und Hyperlipidämie offenbart.

Die Aufgabe der Erfindung besteht insbesondere darin, eine effiziente Ölgewinnung zu erreichen. Die Aufgabe wird erfindungsgemäß durch die Merkmale des Patentanspruchs 1 gelöst, während vorteilhafte Ausgestaltungen und Weiterbildungen der Erfindung den Unteransprüchen und Nebenansprüchen entnommen werden können.

Nicht Gegenstand eines erfindungsgemäßen Verfahrens ist eine Ölgewinnungssubstanz, welche wenigstens einen Bestandteil einer ersten Alge aufweist. Unter einer "Ölgewinnungssubstanz" soll insbesondere eine Substanz verstanden werden, welche dazu vorgesehen ist, mit einer ölhaltigen Substanz in Kontakt gebracht zu werden und Öl der ölhaltigen Substanz von weiteren Bestandteilen der ölhaltigen Substanz, insbesondere in eine eigene Phase, zu separieren. Unter einer "ölhaltigen Substanz" soll insbesondere eine Substanz verstanden werden, welche Öl und/oder Erdöl aufweist. Unter "weiteren Bestandteilen" sollen insbesondere Sand und/oder Bestandteile von Ölsand, welche sich von Öl unterscheiden, und/oder Bestandteile von Bitumen, welche sich von Öl unterscheiden, verstanden werden. Unter einem "Bestandteil" einer Alge soll insbesondere ein Teil der Alge verstanden werden, der sich von Wasser unterscheidet und der wenigstens ein Molekül und/oder wenigstens ein Atom der Alge ist, wobei der Teil vorzugsweise in Wasser löslich ist. Hierdurch kann eine effiziente Ölgewinnung erreicht werden. Insbesondere kann eine effiziente und insbesondere kostengünstige Separierung von Öl der ölhaltigen Substanz von den weiteren Bestandteilen der Substanz erreicht werden. Vorzugsweise ist die Alge eine Salzwasseralge, wodurch eine hohe Wirksamkeit der Ölgewinnungssubstanz erreicht werden kann.

Die Ölgewinnungssubstanz weist wenigstes einen Bestandteil einer zweiten Alge auf, welche einer anderen Sorte angehört als die erste Alge. Auf diese Weise kann eine effektive Zusammensetzung der Ölgewinnungssubstanz erreicht werden.

Die Ölgewinnungssubstanz weist wenigstens einen Bestandteil einer Alge der Sorte Laminaria Digitata und/oder wenigstens einen Bestandteil einer Alge der Sorte Ascophyllum Nodosum und/oder wenigstens einen Bestandteil einer Alge der Sorte Halopteris scolaris auf. Dadurch kann eine wirkungsvolle Zusammensetzung der Ölgewinnungssubstanz erreicht werden. Im Besonderen kann erreicht werden, dass die Ölgewinnungssubstanz wenigstens einen Bestandteil aufweist, welcher zu einer wirkungsvollen Separierung von Öl von den weiteren Bestandteilen geeignet ist.

Erfindungsgemäß wird ein Ölgewinnungssubstanzherstellungsverfahren vorgeschlagen, bei welchem wenigstens ein Teil einer ersten Alge mit zumindest einer weiteren Substanz zusammengebracht wird. Unter einem "Teil" einer Alge, soll insbesondere ein von der Alge abgetrenntes Stück der Alge und insbesondere wenigstens ein Molekül und/oder ein Atom der Alge und insbesondere die gesamte Alge verstanden werden. Dadurch kann eine effiziente Ölgewinnung erreicht werden. Insbesondere kann eine Herstellung einer effizienten Ölgewinnungssubstanz erreicht werden.

Erfindungsgemäß ist die erste Alge von der Sorte Laminaria Digitata. Hierdurch kann eine Herstellung einer besonders effizienten Ölgewinnungssubstanz erreicht werden.

Erfindungsgemäß ist die weitere Substanz zumindest ein Teil einer zweiten Alge, welche einer Sorte angehört, die sich von derjenigen Sorte unterscheidet, welcher die erste Alge angehört, wobei die genannten Teile sich von Wasser unterscheiden. Dadurch kann eine effektive Separierung von Öl erreicht werden.

In einer Ausgestaltung wird vorgeschlagen, dass die zweite Alge von der Sorte Ascophyllum Nodosum ist. Hierdurch kann eine hohe Wirksamkeit einer herzustellenden Ölgewinnungssubstanz zusammen mit einer einfachen Herstellbarkeit erreicht werden.
Mit Vorteil wird der Teil der ersten Alge und der Teil der zweiten Alge mit wenigstens einem Teil einer dritten Alge zusammengebracht, welche einer Sorte angehört, die sich von denjenigen Sorten unterscheidet, welchen die erste Alge und die zweite Alge angehören. Dadurch kann eine effiziente und insbesondere schnelle Herstellung erreicht werden.

Vorzugsweise wird zumindest der Teil der ersten Alge erhitzt. Auf dies Weise kann eine Freisetzung von Stoffen, welche zur Ölseparierung dienen, erreicht werden.

Erfindungsgemäß wird zumindest der Teil der ersten Alge auf im Wesentlichen 95 °C erhitzt. Darunter, dass der Teil der ersten Alge auf "im Wesentlichen 95°C" erhitzt wird, soll insbesondere verstanden werden, dass der Teil der ersten Alge auf eine Temperatur erhitzt wird, welche höchstens 20%, vorzugsweise höchstens 5%, und bevorzugt höchstens 2% kleiner ist all 95°C, wobei die Temperatur besonders bevorzugt genau 95 °C beträgt. Hierdurch kann erreicht werde, dass Bestandteile der ersten Alge, welche zur Ölseparierung dienen, von dem Teil der ersten Alge trennbar und insbesondere in einer Lösung lösbar sind.

Des Weiteren wird eine Ölgewinnungssubstanz vorgeschlagen, welche durch ein Ölgewinnungssubstanzherstellungsverfahren hergestellt wurde. Dadurch kann eine effiziente Ölgewinnung erreicht werden.

Weiterhin wird eine Verwendung einer Ölgewinnungssubstanz vorgeschlagen, bei welcher die Ölgewinnungssubstanz mit einer ölhaltigen Substanz in Kontakt gebracht wird. Dadurch kann eine effiziente Gewinnung von Öl und insbesondere eine effiziente Separierung von Öl der ölhaltigen Substanz von weiteren Bestandteilen der ölhaltigen Substanz erreicht werden.

Ferner wird eine Verwendung einer Ölgewinnungssubstanz vorgeschlagen, bei welcher die Ölgewinnungssubstanz in ein Bohrloch eingebracht wird. Unter einem "Bohrloch" soll insbesondere ein in einen Erdoberflächenbereich eingebrachtes Loch verstanden werden, welches zur Förderung von Öl aus einem erdinneren Raumbereich vorgesehen ist. Dadurch kann eine effiziente Ölgewinnung erreicht werden. Insbesondere kann erreicht werden, dass aus erdinneren Raumbereichen, welche große Mengen von Ölsand und/oder Bitumen aufweisen, Öl von dem Ölsand und/oder dem Bitumen gewonnen werden kann.

Weitere Vorteile ergeben sich aus der folgenden Zeichnungsbeschreibung. In der Zeichnung sind Ausführungsbeispiele der Erfindung dargestellt. Die Zeichnung, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine schematische Darstellung einer Ölgewinnungssubstanz,
- Fig. 2: eine schematische Darstellung eines Ölgewinnungssubstanzherstellungsverfahrens,
- Fig. 3: eine schematische Darstellung einer Verwendung der Ölgewinnungssubstanz,
- Fig. 4: eine schematische Darstellung eines Resultats einer Wechselwirkung der Ölgewinnungssubstanz mit einer ölhaltigen Substanz und
- Fig. 5: eine schematische Darstellung einer weiteren Verwendung der Ölgewinnungssubstanz, wobei die Ölgewinnungssubstanz in ein Bohrloch gepumpt wird.

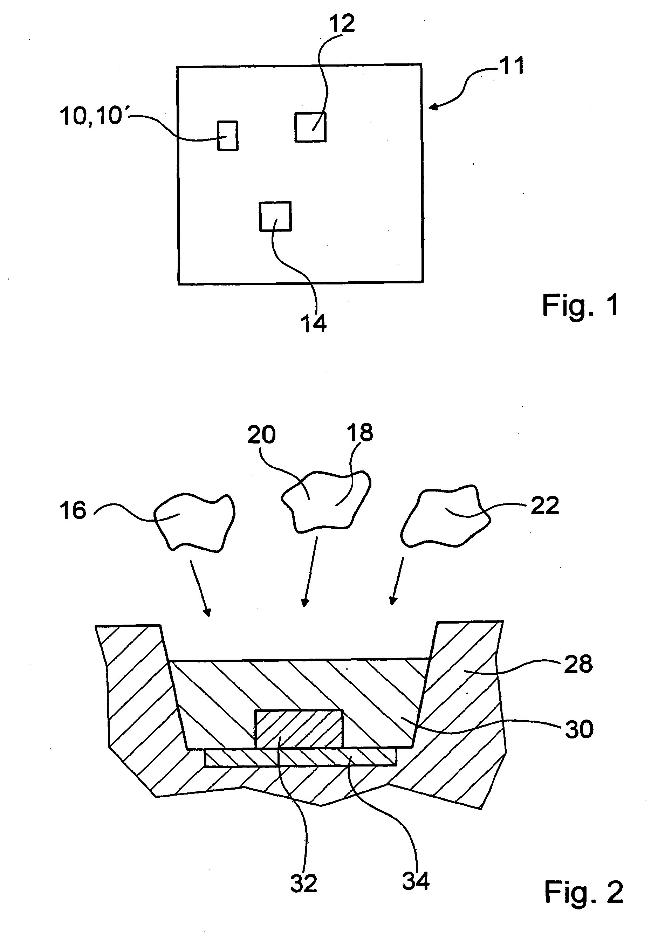

Figur 1 zeigt schematisch eine Ölgewinnungssubstanz 11, welche zumindest bei 20°C eine flüssige Lösung von Bestandteilen 10, 12, 14 von Algen 16, 20, 22 ist. Die Ölgewinnungssubstanz 11 weist die Bestandteile 10 der ersten Alge 16 (Figur 2) auf, welches Enzyme 10', und zwar Lipasen sind. Die erste Alge 16 ist eine Alge 16 der Sorte Laminaria Digitata. Ferner weist die Ölgewinnungssubstanz 11 die Bestandteile 12 der Alge 20, die der Sorte Ascophyllum Nodosum angehört, und die Bestandteile 14 der Alge 22, die der Sorte Halopteris scolaris angehört, auf. Hierbei weisen die Bestandteile 12, 14 Enzyme auf.

Bei einem Ölgewinnungssubstanzherstellungsverfahren, bei welchem die Ölgewinnungssubstanz 11 hergestellt wird, werden Exemplare der Alge 16 mit einer weiteren Substanz 18 zusammengebracht, wobei die Exemplare in Teilchen mit einem maximalen Durchmesser von 280 Mikrometer zerteilt sind. Das Zusammenbringen erfolgt beispielsweise in einem Becken 28 (Figur 2). Die weitere Substanz 18 weist Exemplaren der Alge 20 auf, wobei diese Exemplare in Teilchen mit einem Durchmesser zerteilt sind, der zwischen 500 und 1000 Mikrometern liegt. Ferner werden die Teilchen der Exemplare der Alge 16, 20 mit Exemplaren der Alge 22 zusammengebracht, wobei die Exemplare der Alge 22 in Teilchen zerteilt sind, die kleiner als 500 Mikrometer sind. Außerdem werden die Teilchen der Exemplare der Algen 16, 20, 22 mit Leitungswasser 30 zusammengebracht. Dabei weist das Leitungswasser 30 ein Volumen von 1000 ml auf, die Teilchen der Exemplare der Algen 16 weisen insgesamt eine Masse von 36 Gramm, die Teilchen der Exemplare der Algen 20 weisen insgesamt eine Masse von 48 Gramm und die Teilchen der Exemplare der Algen 22 weisen insgesamt eine Masse von 36 Gramm auf. Das Volumen des Wassers und die Massen können jeweils um zwanzig Prozent größer oder kleiner sein.

Die Gesamtheit der Teilchen der Exemplare der Algen 16, 20, 22 bilden zusammen mit dem Leitungswasser 30 ein Gemisch, das von einem Rührwerk 32 nach dem Zusammenbringen der Teilchen der Exemplare der Algen 16, 20, 22 und des Leitungswassers 30 permanent gerührt wird. Eine Temperatur des Gemischs wird ausgehend von 20°C langsam und kontinuierlich mittels einer Heizung 34 auf 95°C gesteigert. Danach wird das Gemisch für zehn Minuten auf 95°C gehalten. Nachfolgend wird das Gemisch auf 45°C abgekühlt. Anschließend wird das Gemisch auf 45°C für die Zeitdauer von 50 Minuten gehalten. Danach wird das Gemisch auf 20°C abgekühlt. Nach diesem Abkühlen wird das Rühren beendet und mittels Filtration werden feste Rückstände des Gemischs von einem flüssigen Teil des Gemischs getrennt. Die festen Rückstände können bis zu vier Mal für einen Herstellungsprozess, wie er zuvor beschrieben wurde, verwendet werden. Des Weiteren können die festen Rückstände auch auf einer Deponie gelagert oder zu Tierfutter verarbeitet oder als biologisches Düngemittel verwendet werden. Der flüssige Teil des Gemischs ist die Ölgewinnungssubstanz 11.

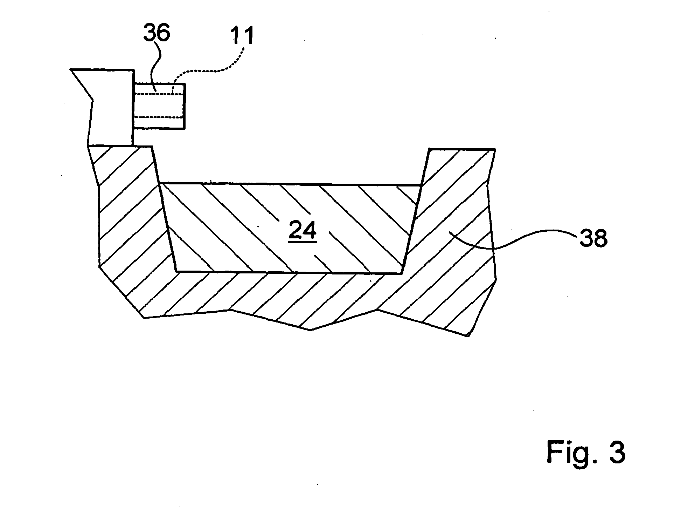

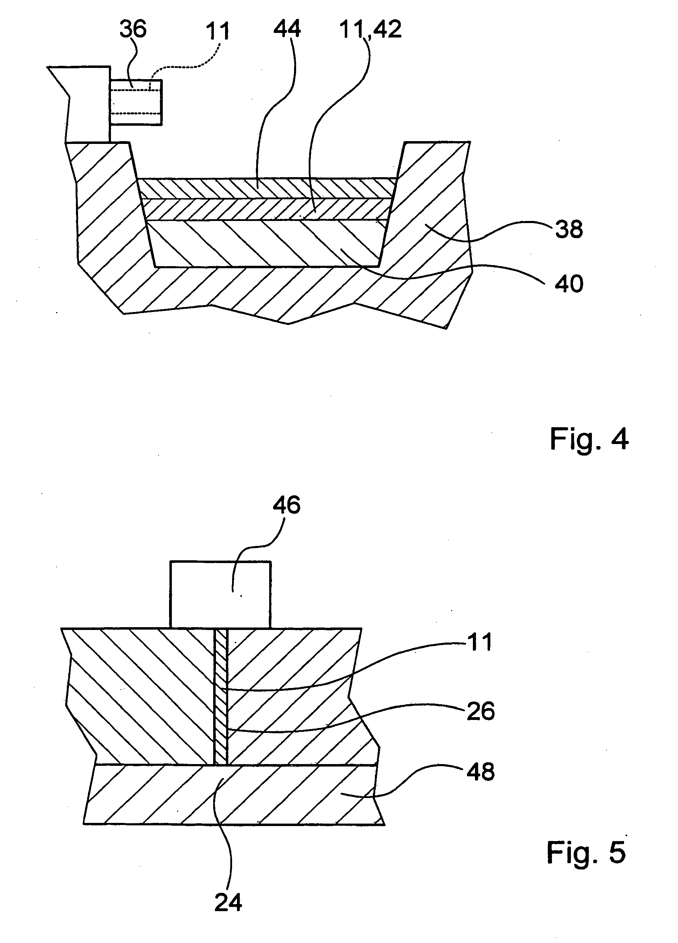

Bei einer Verwendung der Ölgewinnungssubstanz 11 wird diese mit einer ölhaltigen Substand 24, die im beschriebenen Fall Ölsand ist, in Kontakt gebracht (Figur 3). Dabei wird die Ölgewinnungssubstanz 11 mittels einer Leitung 36 in ein Becken 38 einer Kläranlage, in welchem sich die ölhaltigen Substand 24 befindet, eingeleitet. Ein Volumen der ölhaltigen Substand 24 und ein Volumen der Ölgewinnungssubstanz 11, nachdem diese in das Becken eingebracht sind, sind etwa gleich groß. Nachdem die ölhaltigen Substand 24 mit der Ölgewinnungssubstanz 11 wechselgewirkt hat, scheiden sich in dem Becken 38 drei verschiedene Phasen 40, 42, 44 übereinander ab (Figur 4). Die unterste Phase 40 weist im Wesentlichen Sand auf. Die oberste Phase 44 besteht aus Öl. Die mittlere Phase 42 weist die Ölgewinnungssubstanz 11 auf. Ein Abscheiden des Öls wird insbesondere durch Lipasen der Algen 16, 20, 22 bewirkt. Die oberste Phase 44 kann problemlos abgepumpt werden.

Bei einer weiteren Verwendung der Ölgewinnungssubstanz 11 wird die Ölgewinnungssubstanz 11 mittels einer Pumpvorrichtung 46 durch ein Bohrloch 26, welches in einen Erdoberflächenbereich eingebracht ist, in eine Schicht 48 der Erdkruste gepumpt, welche die ölhaltige Substanz 24 aufweist. Die ölhaltige Substanz 24 ist als Ölsand ausgebildet. Wie bei der zuerst beschriebenen Verwendung der Ölgewinnungssubstanz 11 scheidet sich Öl der ölhaltigen Substanz 24 an einem oberen Ende der Schicht 48 ab und kann durch die Pumpvorrichtung 46 abgepumpt werden. Durch die beschriebene Verwendung kann dasjenige Öl, das in der Schicht 48 vor der Verwendung vorhanden ist, fast vollständig separiert werden.

Prinzipiell ist denkbar, zusätzlich zu der Ölgewinnungssubstanz 11 Heißdampf in das Bohrloch 26 einzubringen und zu der Schicht 48 zu bringen, welcher für eine Separierung des Öls förderlich ist.

Eine weitere Verwendung der Ölgewinnungssubstanz 11 besteht darin, einen ölverschmutzten Landschaftsbereich mit der Ölgewinnungssubstanz 11 in Kontakt zu bringen. Dabei bewirkt die Ölgewinnungssubstanz 11, dass der ölverschmutzte Landschaftsbereich in einer eigenen Phase abgetrennt wird. Dadurch wird der Landschaftsbereich gereinigt.

### Bezugszeichen

- 10: Bestandteil
- 10': Enzym
- 11: Ölgewinnungssubstanz
- 12: Bestandteil
- 14: Bestandteil
- 16: Alge
- 18: Substanz
- 20: Alge
- 22: Alge
- 24: Substanz
- 26: Bohrloch
- 28: Becken
- 30: Leitungswasser
- 32: Rührwerk
- 34: Heizung
- 36: Leitung
- 38: Becken
- 40: Phase
- 42: Phase
- 44: Phase
- 46: Pumpvorrichtung
- 48: Schicht

## Patentansprüche

1. Ölgewinnungssubstanzherstellungsverfahren bei welchem wenigstens ein Teil einer ersten Alge (16) mit zumindest einer weiteren Substanz (18) zusammengebracht wird, wobei die erste Alge (16) von der Sorte Laminaria Digitata ist und wobei die weitere Substanz (18) zumindest ein Teil einer zweiten Alge (20) ist, welche einer Sorte angehört, die sich von derjenigen Sorte unterscheidet, welche die erste Alge (16) angehört, wobei die genannten Teile sich von Wasser unterscheiden und wobei zumindest der Teil der ersten Alge (16) auf im Wesentlichen 95°C erhitzt wird.

2. Ölgewinnungssubstanzherstellungsverfahren nach Anspruch 1,
**dadurch gekennzeichnet**, **dass**
die zweite Alge (20) von der Sorte Ascophyllum Nodosum ist.

3. Ölgewinnungssubstanzherstellungsverfahren nach einem der Ansprüche 1 oder 2,
**dadurch gekennzeichnet**, **dass**
der Teil der ersten Alge (16) und der Teil der zweiten Alge (20) mit wenigstens einem Teil einer dritten Alge (22) zusammengebracht wird, welche einer Sorte angehört, die sich von denjenigen Sorten unterscheidet, welchen die erste Alge (16) und die zweite Alge (20) angehören.

4. Ölgewinnungssubstanz, welche durch ein Ölgewinnungssubstanzherstellungsverfahren nach einem der Ansprüche 1 bis 3 hergestellt wurde.

5. Verwendung einer Ölgewinnungssubstanz nach Anspruch 4, bei welcher die Ölgewinnungssubstanz mit einer ölhaltigen Substanz (24) in Kontakt gebracht wird.

6. Verwendung einer Ölgewinnungssubstanz nach Anspruch 5, bei welcher die Ölgewinnungssubstanz in ein Bohrloch (26) eingebracht wird.

## Claims

1. Method to produce a substance for obtaining oil, in which at least one component of a first alga (16) is brought together with at least one further substance (18), wherein the first alga (16) is of the species Laminaria Digitata and wherein the further substance (18) is at least one component of a second alga (20) which belongs to a species that differs from the species the first alga (16) belongs to, wherein said components differ from water and wherein at least the component of the first alga (16) is heated to substantially 95°C.

2. Method to produce a substance for obtaining oil according to claim 1,
**characterized in that**
the second alga (20) is of the species Ascophyllum Nodosum.

3. Method to produce a substance for obtaining oil according to one of claims 1 or 2,
**characterized in that**
the component of the first alga (16) and the component of the second alga (20) are brought together with at least one component of a third alga (22) which belongs to a species that differs from the species which the first alga (16) and the second alga (20) belong to.

4. Substance for obtaining oil, which has been produced by a method to produce a substance for obtaining oil according to one of claims 1 to 3.

5. Usage of a substance for obtaining oil according to claim 4, in which the substance for obtaining oil is brought into contact with an oil-containing substance (24).

6. Usage of a substance for obtaining oil according to claim 5, in which the substance for obtaining oil is introduced into a drill hole.

## Revendications

1. Méthode pour produire une substance prévue à obtenir de l'huile, dans laquelle méthode au moins un composant d'une première algue (16) est ramené en contact avec au moins une substance (18) de plus,
la première algue (16) étant de l'espèce Laminaria Digitata et la substance (18) de plus étant au moins un composant d'une deuxième algue (20), laquelle appartient à une espèce différente de l'espèce à laquelle appartient la première algue (16),
lesdits composants étant différents de l'eau et au moins le composant de la première algue étant chauffé jusqu'à environ 95°C.

2. Méthode pour produire une substance prévue à obtenir de l'huile selon la revendication 1,
**caractérisée en ce que**
la deuxième algue (20) est de l'espèce Ascophyllum Nodosum.

3. Méthode pour produire une substance prévue à obtenir de l'huile selon une des revendications 1 ou 2,
**caractérisée en ce que**
le composant de la première algue (16) et le composant de la deuxième algue (20) sont ramenés avec au moins un composant d'une troisième algue (22), laquelle appartient à une espèce différente des espèces à lesquelles appartiennent la première algue (16) et la deuxième algue (20).

4. Substance prévue à obtenir de l'huile, produite par la biais d'une méthode pour produire une substance prévue à obtenir de l'huile selon l'une quelconque des revendications 1 à 3.

5. Usage d'une substance prévue à obtenir de l'huile selon la revendication 4, dans lequel la substance prévue à obtenir de l'huile est ramenée en contact avec une substance (24) oléagineuse.

6. Usage d'une substance prévue à obtenir de l'huile selon la revendication 5, dans lequel la substance prévue à obtenir de l'huile est introduite dans un puits (26).
